# EUROPÄISCHE PATENTSCHRIFT

(11) **EP 3 720 815 B1**
(45) Veröffentlichungstag und Bekanntmachung des Hinweises auf die Patenterteilung: **05.01.2022**
(21) Anmeldenummer: 18812145.3
(22) Anmeldetag: 30.11.2018
(51) Int. Cl.: C01C 1/04, B01D 53/14, B01D 53/62, B01D 53/78, C07C 273/04, C07C 273/10

(54) **VERFAHREN ZUR BEREITSTELLUNG VON CO2 FÜR DIE HARNSTOFFSYNTHESE AUS RAUCH- UND SYNTHESEGAS**
METHOD FOR PROVIDING CO2 FOR SYNTHESIZING UREA FROM FLUE GAS AND SYNGAS
PROCÉDÉ POUR LA FOURNITURE DE CO2 POUR LA SYNTHÈSE DE L'URÉE À PARTIR DE FUMÉES ET DE GAZ DE SYNTHÈSE

(30) Priorität: 06.12.2017 DE 102017222030
(43) Veröffentlichungstag der Anmeldung: 14.10.2020
(73) Patentinhaber: thyssenkrupp Industrial Solutions AG, 45143 Essen (DE); thyssenkrupp AG, 45143 Essen (DE)
(72) Erfinder: MAKHYNYA, Yevgeny, 45481 Mülheim an der Ruhr (DE); EL-HAWARY, Tarek, 59439 Holzwickede (DE)
(74) Vertreter: thyssenkrupp Intellectual Property GmbH
(86) Internationale Anmeldenummer: PCT/EP2018/083137
(87) Internationale Veröffentlichungsnummer: WO 2019/110443

(56) Entgegenhaltungen:
- US-A1- 2007 287 863
- US-A1- 2013 174 566
- US-A1- 2014 123 850
- US-B2- 9 428 449

## Beschreibung

Die vorliegende Erfindung betrifft ein Verfahren zur Bereitstellung von Kohlendioxid auf hohem Druck- und Temperaturniveau für die Synthese von Harnstoff aus Ammoniak und Kohlendioxid, bei dem als ein erstes Ausgangsgasgemisch ein Kohlendioxid-haltiges Rauchgas verwendet wird, wobei dieses erste Ausgangsgasgemisch einem Niederdruckabsorber zugeführt wird, in dem Kohlendioxid aus dem ersten Ausgangsgasgemisch in einem flüssigen Lösungsmittel auf Basis eines Ammoniak-Wasser-Gemisches absorbiert wird, wobei weiterhin als ein zweites Ausgangsgasgemisch ein Kohlendioxid-haltiges Synthesegas verwendet wird und dieses zweite Ausgangsgasgemisch einem Hochdruckabsorber zugeführt wird, in dem Kohlendioxid aus diesem zweiten Ausgangsgasgemisch in einem flüssigen Lösungsmittel auf Basis eines Ammoniak-Wasser-Gemisches bei erhöhtem Druck absorbiert wird.

Die großtechnische Herstellung von Harnstoff beruht derzeit praktisch ausschließlich auf der Hochdrucksynthese aus Ammoniak und Kohlendioxid in einer Harnstoff-Anlage bei einem Druck von ungefähr 150 bar und einer Temperatur von ungefähr 180 °C.

Beide Einsatzstoffe für die Harnstoffsynthese werden in der Regel in einer Ammoniak-Anlage bereitgestellt, welche meist in direkter Nachbarschaft zu der betreffenden Harnstoff-Anlage steht. Da das Ammoniak an der Anlagengrenze der Ammoniak-Anlage in der Regel flüssig vorliegt, kann es mit einem begrenzten energetischen und apparativen Aufwand auf das Druckniveau der Harnstoff-Synthese gebracht werden. Das Kohlendioxid fällt in der Ammoniak-Anlage jedoch gasförmig an und es wird daher ein ungleich größerer energetischer und apparativer Aufwand benötigt, um das Kohlendioxid auf das Druckniveau der Harnstoffanlage zu bringen.

Im Regelfall produziert eine herkömmliche Ammoniak-Anlage etwa 10 % mehr Ammoniak, als für eine stöchiometrische Umsetzung des Ammoniaks mit dem Kohlendioxid zu Harnstoff notwendig wäre. Wenn hingegen das gesamte anfallende Ammoniak und das dazu benötigte stöchiometrische Kohlendioxid zu Harnstoff umgesetzt werden, spricht man von einer so genannten "balanced plant". Für die Konstruktion einer solchen "balanced plant" sind im Vergleich zum klassischen Ammoniak-Harnstoff-Anlagenkomplex weitere apparative und verfahrenstechnische Maßnahmen erforderlich.

Bei der Erneuerung und Modernisierung bestehender Anlagen (so genanntes "Revamp") sowie auch bei der Konstruktion von "balanced plant" Anlagen wird oft eine Quelle für zusätzliches Kohlendioxid benötigt. Dieses Kohlendioxid ist zum Beispiel aus dem Rauchgas erhältlich. Rauchgase fallen jedoch bei Atmosphärendruck an und dort abgetrenntes Kohlendioxid muss energetisch aufwändig auf den Druck der Harnstoffsynthese gebracht werden. Der apparative Aufwand hierfür ist sehr hoch, insbesondere wenn große zusätzliche Mengen an Kohlendioxid benötigt werden. Bei Revamp-Maßnahmen an einer bestehenden Anlage sind die kritischsten Anlagenbereiche zumeist die CO₂-Wäsche und der CO₂-Kompressor.

Das für die Harnstoff-Synthese benötigte Kohlendioxid fällt in der Synthesegas-Erzeugung einer Ammoniakanlage als Bestandteil des Rohsynthesegases nach der Reformierung an. Da das Kohlendioxid in der Ammoniaksynthese als Katalysatorgift wirken würde, muss es aus dem Synthesegas abgetrennt werden. Nach dem Stand der Technik geschieht dies in der Regel durch Einsatz regenerativer Gaswäschen, wobei eine größere Anzahl selektiv wirkender Lösungsmittel bekannt sind, die für diese Gaswäsche verwendbar sind.

Ebenfalls bekannt ist es, Kohlendioxid aus Rauchgasen durch regenerative Gaswäsche mit selektiv wirkenden Lösungsmitteln oder mit Ammoniak oder Ammoniak-Wasser-Gemischen abzutrennen. Im letztgenannten Fall wird das Kohlendioxid in der Lösung vorwiegend chemisch in Form von Karbamat und Karbonat-Ionen gebunden. Die beladene Lösung wird thermisch regeneriert, wobei das Kohlendioxid drucklos oder nahezu drucklos anfällt. Detaillierte Untersuchungen des Anmelders haben jedoch gezeigt, dass die Durchführung von Gaswäschen mit einem Ammoniak-Wasser-Gemisch bei diesen Bedingungen keine energetischen Vorteile gegenüber Wäschen mit selektiv wirkenden Lösungsmitteln wie beispielsweise Alkanolaminen hat.

Aus der DE 10 2015 121 756 A1 ist ein Verfahren zur Herstellung von Harnstoff bekannt, bei dem ein Gasstrom umfassend Wasserstoff, Stickstoff und Kohlendioxid bereitgestellt wird, zumindest ein Teil des Kohlendioxids mit einem Lösungsmittel aus diesem Gasstrom abgetrennt wird, Ammoniak aus zumindest einem Teil des Wasserstoffs und zumindest einem Teil des Stickstoffs, welche in dem Kohlendioxid-abgereicherten Gasstrom enthalten sind, synthetisiert wird, das Kohlendioxid aus dem Lösungsmittel desorbiert wird und Harnstoff aus dem zuvor synthetisierten Ammoniak und dem zuvor desorbierten Kohlendioxid hergestellt wird. Bei diesem bekannten Verfahren handelt es sich bei dem Ausgangsgasgemisch, welches Wasserstoff, Stickstoff und Kohlendioxid enthält, um ein Synthesegas, welches durch Dampfreformierung oder durch autotherme Reformierung erzeugt wird. Die Abtrennung des Kohlendioxids erfolgt bei diesem Verfahren durch eine Ammoniak-Wasser-Wäsche. Das die Abtrennvorrichtung verlassende Kohlendioxid-angereicherte Lösungsmittel wird komprimiert auf einen Zieldruck oberhalb des Harnstoffsynthesedrucks und das komprimierte Lösungsmittel wird einer Desorptionsvorrichtung zugeführt, in welcher das Kohlendioxid aus dem Lösungsmittelstrom desorbiert wird, bei einem Druck, welcher oberhalb des Betriebsdrucks der Harnstoffsyntheseeinheit liegt. Das in der Desorptionsvorrichtung desorbierte Kohlendioxid wird in die Harnstoffsyntheseeinheit geleitet und dort mit dem Ammoniak unter Bildung von Harnstoff umgesetzt.

In der US 9,428,449 B2 wird eine integrierte Ammoniak- und Harnstoffsynthese beschrieben, bei der ein in einem Reformer erzeugtes Synthesegas zunächst einer CO-Shift-Reaktion unterworfen wird und das erhaltene Gemisch aus Wasserstoff und Kohlendioxid einem Hochdruckabsorber zugeführt wird, in dem eine Gaswäsche mit einem halbfetten Lösungsmittelstrom erfolgt. In dem Reformer fällt außerdem ein Rauchgasstrom an, welcher einem Niederdruckabsorber zugeführt wird, in dem eine Gaswäsche mit einem abgereicherten (mageren) Lösungsmittel erfolgt, wobei ein Stickstoff-haltiger Strom erhalten wird, der einem Ammoniaksynthesereaktor zugeführt wird. Durch die Gaswäsche des Synthesegases in dem Hochdruckabsorber wird das Kohlendioxid aus diesem Gasstrom entfernt und ein Wasserstoffhaltiger Strom erhalten, der ebenfalls dem Ammoniaksynthesereaktor zugeführt wird, wobei das dort synthetisierte Ammoniak anschließend einem Harnstoffsynthesereaktor zugeführt wird. Für dieses bekannte Verfahren kann man den Begriff "integrierte Ammoniak- und Harnstoffsynthese" verwenden, weil das in dem Anlagenkomplex hergestellte Ammoniak sofort für die CO₂-Abtrennung verwendet wird, das Reaktionsprodukt--Ammoniumcarbamat--ein Zwischenprodukt der Harnstoffsynthese ist und dieses Zwischenprodukt sofort für die Harnstoffsynthese verwendet wird. Bei diesem bekannten Verfahren wird als mageres Lösungsmittel ein Recyclestrom aus der Harnstoffanlage verwendet, welcher Ammoniumkarbamat, Ammoniumcarbonat, Ammoniumbicarbonat in Lösung oder als Aufschlämmung enthält, sowie weiterhin nicht reagiertes Ammoniak, Kohlendioxid und Wasser, wobei diesem Strom gegebenenfalls weiterer Ammoniak zugegeben wird. Dieses magere Lösungsmittel wird dann zunächst dem Niederdruckabsorber zugeführt, in dem Kohlendioxid aus dem Rauchgas absorbiert wird, so dass ein an Kohlendioxid angereichertes Lösungsmittel erhalten wird, welches danach dem Hochdruckabsorber zugeführt wird, in dem weiteres Kohlendioxid aus dem Synthesegas absorbiert wird. Das stark angereicherte Lösungsmittel aus dem Hochdruckabsorber, welches neben Kohlendioxid Ammoniak und Wasser enthält, wird dann der Harnstoffsynthese zugeführt. Das an Kohlendioxid abgereicherte Lösungsmittel aus dem Harnstoffsynthesereaktor wird dann wieder als mageres Lösungsmittel zum Niederdruckabsorber geleitet.

Es ist typisch für eine integrierte Synthese, wie sie im US-Patent 9,428,449 B2 beschrieben wird, bei der ein Ammoniaksynthesereaktor und ein Harnstoffsynthesereaktor in ein Anlagenkonzept eingebunden sind, dass CO₂-haltige Gase mit Ammoniak oder mit sehr konzentrierter Ammoniak-Lösung in Wasser in Kontakt gebracht werden. In Abwesenheit von Wasser entsteht überwiegend Ammoniumkarbamat, welches direkt in die Harnstoff-Synthese weitergeleitet wird. Es ist offensichtlich, dass dies nur bei sehr geringen Mengen Wasser in der Waschlösung funktionieren kann, da das Wasser sowohl für Karbamat als auch für die Harnstoff-Synthese unerwünscht ist. Eine weitere Besonderheit von integrierten Prozessen dieser Art, wie sie aus dem Stand der Technik bekannt sind, ist die Tatsache, dass beide Anlagenteile (für die Ammoniak- und die Harnstoffsynthese) nur zusammen betrieben werden können, was den Betrieb unflexibel macht und die Start-ups und die Shut-downs der Anlagenteile erheblich erschwert. Aus diesem Grunde wurde vermutlich bislang noch keine integrierte Anlage in industrieller Größe gebaut.

Die Aufgabe der vorliegenden Erfindung besteht darin, ein Verfahren zur Bereitstellung von Kohlendioxid für die Synthese von Harnstoff aus Ammoniak und Kohlendioxid mit den Merkmalen der eingangs genannten Gattung zur Verfügung zu stellen, welches im Vergleich zu bekannten Verfahren einen geringeren spezifischen Energieverbrauch und reduzierte Kapitalkosten aufweist.

Die Lösung der vorgenannten Aufgabe liefert ein Verfahren der eingangs genannten Art mit den Merkmalen des Anspruchs 1.

Erfindungsgemäß ist vorgesehen, dass für den Absorptionsschritt in dem Niederdruckabsorber und für den Absorptionsschritt in dem Hochdruckabsorber das gleiche an Kohlendioxid abgereicherte flüssige Lösungsmittel verwendet wird, welches in wenigstens zwei parallelen Teilströmen dem Hochdruckabsorber und dem Niederdruckabsorber zugeführt wird.

Anders als bei dem aus der US-Schrift 9,428,449 bekannten Verfahren erfolgt erfindungsgemäß der Waschvorgang zur Entfernung des Kohlendioxids aus dem Gasstrom nicht sukzessive, so dass eine bereits teilweise an Kohlendioxid angereicherte Lösung aus dem Niederdruckabsorber in den Hochdruckabsorber gelangt, sondern der Waschvorgang erfolgt parallel mit der gleichen an Kohlendioxid abgereicherten Waschlösung, die dazu in zwei Teilströme aufgeteilt wird, von denen der eine dem Niederdruckabsorber zugeführt wird und der andere dem Hochdruckabsorber zugeführt wird. Der Ausdruck "gleichen an Kohlendioxid abgereicherten Waschlösung" umfasst bevorzugt im Sinne der Erfindung auch eine geringfüge Abweichung in der Zusammensetzung der beiden Teilströme. Physikalische Prozesse, beispielsweise Ausgasung gelöster Gase, können Druckentspannungen erforderlich machen. Es werden bevorzugt gleiche an Kohlendioxid abgereicherte Waschlösung verwendet, die geringfügigen Abweichungen in Zusammensetzung und Temperatur aufweisen können. Diese Abweichungen sind bevorzugt durch die weitere zwangsläufige Entspannung (und dadurch Energierückgewinnung) auf Druckniveau des Niederdruckabsorbers zurückzuführen. Bevorzugt bleiben bei einer Druckentspannung (beispielsweise von 36 bar auf 1 bar) die Hauptkomponenten CO₂, NH₃ und H₂O nahezu unverändert (+/- 10 %). Lediglich geringe Anteile physikalisch gelöste Gase, vor allem H₂ und N₂, entweichen. Dies hat den Vorteil, dass eine abgereicherte Waschlösung anstelle einer schon teilweise mit Kohlendioxid beladenen Lösung in den Hochdruckabsorber gelangt.

Gemäß einer bevorzugten Weiterbildung des erfindungsgemäßen Verfahrens werden nach der Kohlendioxidwäsche der erste, aus dem Niederdruckabsorber austretende, mit Kohlendioxid angereicherte Lösungsmittelstrom und der zweite, aus dem Hochdruckabsorber austretende, mit Kohlendioxid angereicherte Lösungsmittelstrom gegebenenfalls nach dem Entfernen von Inerten anschließend zu einem gemeinsamen Lösungsmittelstrom vereint.

Anders als im Stand der Technik wird jedoch dieser mit Kohlendioxid angereicherte Lösungsmittelstrom aus den beiden Absorbern nicht der Harnstoffanlage zugeleitet, sondern die Lösung wird auf hohen Druck gebracht und das Kohlendioxid wird aus dem Lösungsmittel ausgetrieben. Vorzugsweise wird dabei das Kohlendioxid entweder aus einem ersten, aus dem Niederdruckabsorber austretenden mit Kohlendioxid angereicherten Lösungsmittelstrom oder aus einem zweiten, aus dem Hochdruckabsorber austretenden mit Kohlendioxid angereicherten Lösungsmittelstrom oder aus einem vereinten gemeinsamen Lösungsmittelstrom aus Niederdruckabsorber und Hochdruckabsorber anschließend in einer Desorptionsvorrichtung desorbiert.

Untersuchungen des Anmelders haben gezeigt, dass die Ammoniak-Wasser-Wäsche von Rauchgas und Synthesegas in den beiden Absorbern so betrieben werden kann, dass sowohl eine Verbesserung des spezifischen Energieverbrauchs als auch eine Reduzierung der Kapitalkosten möglich ist. Erreicht werden diese Vorteile insbesondere, indem das Kohlendioxid aus dem Lösungsmittel nicht wie allgemein üblich bei einem verhältnismäßig niedrigen Druck ausgetrieben wird, sondern bei einem Druckniveau bevorzugt leicht oberhalb des Harnstoffsynthesedrucks, so dass das desorbierte Kohlendioxid anschließend problemlos in die Harnstoffsynthese eingebracht werden kann und das ohne weitere Kompressionsarbeit. Anders als im Stand der Technik wird somit nicht eine mit Kohlendioxid beladene Waschlösung (rich solvent) der Harnstoffsynthese zugeführt, sondern das aus der Waschlösung desorbierte Kohlendioxid. Die abgereicherte Lösung nach dem Desorptionsvorgang kann bevorzugt als Waschlösung zu den beiden Absorbern zurückgeführt und somit in dem Absorptionsprozess zur Entfernung des Kohlendioxids erneut genutzt werden. Damit ist beispielsweise ein CO₂-Kompressor ist nicht mehr notwendig.

Gemäß einer bevorzugten Weiterbildung des Verfahrens wird das mit Kohlendioxid angereicherte Lösungsmittel in der Desorptionsvorrichtung bei erhöhter Temperatur aus dem Lösungsmittel desorbiert, vorzugsweise bei einer Temperatur von wenigstens 150 °C, besonders bevorzugt bei einer Temperatur von wenigstens 180 °C, beispielsweise bei etwa 200 °C. Ein erheblicher Vorteil der Kohlendioxidwäsche, wie sie in dem erfindungsgemäßen Verfahren durchgeführt wird, liegt darin, dass das Kohlendioxid zwangsläufig auf hohem Druck und über eine thermische Regeneration im Desorber bei relativ hoher Temperatur bereitgestellt wird. Durch diese Maßnahmen kann Kohlendioxid auf einem Temperatur- und Druckniveau bereitgestellt werden, welches gerade für die Harnstoffsynthese optimal ist und keine weiteren verfahrenstechnische Schritte für die Anpassung von Druck und Temperatur erfordert.

Lediglich braucht man einen etwas höheren Druck um die Druckverluste auf dem Weg zur Harnstoffanlage zu überwinden. Aus der Thermodynamik ist bekannt, dass bei der Destillation in ternären Systemen die Druckerhöhung faktisch dazu führt, dass die leichtflüchtigste Komponente aus der Lösung "rausgedrückt" wird, also dass der Partialdruck der leichtesten Komponente über dem Gemisch erhöht wird. In dem ternären Gemisch NH₃-CO₂-H₂O ist das CO₂ die leichtflüchtigste Komponente. Deswegen ist eine Druckerhöhung in dem erfindungsgemäßen Verfahren nach der Absorption und Entspannung besonders vorteilhaft.

Besonders vorteilhaft ist es, wenn diese Druckerhöhung gemäß einer bevorzugten Ausgestaltung des erfindungsgemäßen Verfahrens mittels einer Pumpe im flüssigen Zustand durchgeführt wird, wodurch man einen CO₂-Kompressor und die dazugehörige Peripherie einspart. Durch diese Maßnahme werden sowohl die Energieverbrauchskosten als auch die Kapitalkosten der Anlage gesenkt.

Vorzugsweise geht man dabei so vor, dass man das mit Kohlendioxid angereicherte Lösungsmittel aus dem Hochdruckabsorber zunächst abkühlt und danach entspannt, wobei in dem Lösungsmittel absorbierte Gase, die von Kohlendioxid verschieden sind (Inerte), insbesondere Stickstoff und/oder Wasserstoff aus dem Lösungsmittel ausgegast und abgetrennt werden. Anschließend vereint man das mit Kohlendioxid angereicherte Lösungsmittel aus dem Hochdruckabsorber mit dem angereicherten Lösungsmittel aus dem Niederdruckabsorber und erhöht vorzugsweise danach den Druck des vereinten Lösungsmittelstroms, bevor man diesen der Desorptionsvorrichtung zuführt.

Untersuchungen im Rahmen der vorliegenden Erfindung haben gezeigt, dass das Kohlendioxid mit der erforderlichen Reinheit bei einem noch moderaten Temperaturniveau aus dem Lösungsmittel extrahiert werden kann. Das benötigte Temperaturniveau ist aber mit beispielsweise etwa 200 °C noch so hoch, dass herkömmliche selektiv wirkende Lösungsmittel, die sonst bei der Gaswäsche verwendet werden, wie zum Beispiel Alkanolamine, wegen ihrer begrenzten thermischen Stabilität nicht verwendet werden können. Wegen des relativ hohen Dampfdrucks bereits bei mäßigen Temperaturen und der verhältnismäßig großen Desorptionswärme kommt Ammoniak als reines Lösungsmittel für die Gaswäsche nicht in Betracht. Auch kann man reines Ammoniak als Waschmittel nicht regenerieren und recyclieren, so dass das Ammoniak verbraucht werden müsste. Ammoniak würde in diesem Fall zu Karbamat führen, welches man dann der Harnstoffsynthese zuführen müsste. Damit würde sich im Prinzip wieder ein "integriertes" Verfahren ergeben, welches die vorliegende Erfindung vermeiden will.

Deshalb wird in dem erfindungsgemäßen Verfahren vorgeschlagen, für die CO₂-Wäsche auf Ammoniak-Wasser-Basis in den beiden Absorbern eine verdünnte Ammoniak-Lösung zu verwenden, insbesondere eine wässrige Ammoniaklösung mit einer Konzentration von vorzugsweise etwa 15-25 Gew.-%. Das in der Desorptionsvorrichtung ausgetriebene Kohlendioxid kann für die Harnstoffsynthese bereitgestellt werden. Das erfindungsgemäße Verfahren entfaltet sein volles Potential bei Einsatz in einem Ammoniak-Harnstoff-Komplex. Es ist aber auch möglich, das erfindungsgemäße Verfahren bei einer allein stehenden Ammoniak-Anlage einzusetzen. Hier zeigt sich die hohe Flexibilität des Verfahrens.

Gemäß der vorliegenden Erfindung wird die Gaswäsche in beiden Absorbern mit der gleichen Ammoniak-Wasser-Lösung durchgeführt, um einerseits Kohlendioxid aus Synthesegas und andererseits Kohlendioxid aus Rauchgas zu binden. Gemäß einer bevorzugten Variante des Verfahrens wird dabei eine Apparatur verwendet, welche beispielsweise in zwei Zonen unterteilt sein kann (was aber nicht zwingend ist), nämlich einen Hochdruckabsorber und einen bei atmosphärischem Druck arbeitenden Absorber. Folglich muss die mit Kohlendioxid beladene Lösung aus dem Hochdruckabsorber anschließend entspannt werden, um sie auf einen Druck von beispielsweise etwa 2 bar zu bringen, wobei in der Waschlösung ebenfalls absorbierte für die Harnstoffsynthese inerte Gase wie insbesondere Wasserstoff und Stickstoff freigesetzt werden. Die mit Kohlendioxid beladene Waschlösung aus dem Niederdruckabsorber (atmosphärischen Absorber) muss hingegen auf einen etwas höheren Druck von beispielsweise 2 bar gebracht werden und danach kann man die beiden Stoffströme vereinigen.

Anschließend wird die vereinigte Waschlösung vorzugsweise mittels einer oder mehrerer Pumpen auf einen Druck gebracht, der leicht oberhalb des Harnstoffsynthesedrucks liegt und die Waschlösung wird in der Desorptionsvorrichtung thermisch regeneriert.

Vorzugsweise wird von Kohlendioxid abgereichertes Lösungsmittel aus der Desorptionsvorrichtung wenigstens teilweise zu dem Niederdruckabsorber und/oder wenigstens teilweise zu dem Hochdruckabsorber zurückgeführt.

Gemäß einer Weiterbildung des Verfahrens wird vorzugsweise von Kohlendioxid abgereichertes Lösungsmittel aus der Desorptionsvorrichtung bei der Rückführung zunächst in wenigstens einer ersten Turbine unter Nutzung der gespeicherten Energie auf einen niedrigeren Druck entspannt und ein Teilstrom dieses Lösungsmittels wird zu dem Hochdruckabsorber zurückgeführt und ein weiterer Teilstrom dieses Lösungsmittels wird in wenigstens einer zweiten Turbine unter Nutzung der gespeicherten Energie auf einen noch niedrigeren Druck entspannt und danach mindestens teilweise zu dem Niederdruckabsorber zurückgeführt.

Weiterhin kann es vorteilhaft sein, wenn der vereinte Lösungsmittelstrom aus dem Niederdruckabsorber und dem Hochdruckabsorber, bevor dieser der Desorptionsvorrichtung zugeführt wird, in wenigstens zwei Teilströme zwecks der Wärmeintegration aufgeteilt wird, von denen der erste Teilstrom einem ersten Wärmetauscher zugeführt wird und der zweite Teilstrom einem zweiten Wärmetauscher zugeführt wird, wobei nach Durchströmen der beiden Wärmetauscher die beiden Teilströme wieder vereint und dann der Desorptionsvorrichtung zugeführt werden.

Eine bevorzugte Weiterbildung des Verfahrens sieht eine Aufbereitung des Rauchgasstroms vor, bevor dieser dem Niederdruckabsorber zugeführt wird. Dazu kann beispielsweise das Rauchgas vor dem Schritt der Absorption in dem Niederdruckabsorber erwärmt oder gekühlt werden. Oder das Rauchgas wird vor dem Schritt der Absorption in dem Niederdruckabsorber zur Entfernung von Flugasche oder Rußpartikeln filtriert oder einer Nasswäsche unterzogen.

Das erfindungsgemäße Verfahren ist anwendbar sowohl bei der Konstruktion neuer Ammoniak-Harnstoff-Komplexe als so genannte "balanced plant" als auch bei der Kapazitätserweiterung einer existierenden Harnstoff-Anlage oder bei Kapazitätserweiterung eines existierenden Ammoniak-Harnstoff-Komplexes im Falle einer geringfügigen Kapazitätserweiterung der Ammoniak-Anlage oder einer bedeutenden Kapazitätserweiterung der Harnstoff-Anlage sowie auch im Falle einer bedeutenden Kapazitätserweiterung von Ammoniak- und Harnstoffanlage. Unter einer geringfügigen Erweiterung der Kapazität wird in diesem Zusammenhang eine Erweiterung von beispielsweise etwa 5 % bis etwa 10 % verstanden und unter einer bedeutenden Erweiterung der Kapazität wird eine solche von beispielsweise etwa 10 % bis etwa 30 % und mehr verstanden. In den Fällen einer Kapazitätserweiterung ist es zu empfehlen, die Ammoniak-Wasser-Wäsche in den beiden Absorbern parallel zu einer bereits in der Anlage existierenden Wäsche zu betreiben, unabhängig davon, welcher Art diese Wäsche ist.

Die für die Desorption des Kohlendioxids benötigte Wärmemenge kann bei beiden vorgenannten Varianten- Neuanlage oder Kapazitätserweiterung- mit begrenzten Modifikationen des Dampfsystems in einem Ammoniak-Harnstoffkomplex verfügbar gemacht werden. Da der Dampfbedarf für den Antrieb des CO₂-Verdichters entfällt, ergibt sich insbesondere bei einer neuen Anlage eine signifikante Reduzierung des Energiebedarfs, der sich beispielsweise in einer reduzierten Feuerungsleistung eines vorhandenen Hilfskessels widerspiegeln kann. Im Falle einer Kapazitätserweiterung kann die Energieersparnis entsprechend geringer sein. Jedoch wird der CO₂-Verdichter nicht zusätzlich beansprucht, bzw. bei größeren Kapazitätssteigerungen ist kein zusätzlicher CO₂-Verdichter notwendig. Mit dem erfindungsgemäßen Verfahren kann man virtuell beliebige zusätzliche CO₂-Mengen für eine Harnstoffsynthese zur Verfügung stellen.

Die größte Reduzierung der Anlageninvestitionskosten ist im Falle einer neuen Anlage zu erwarten, da der Wegfall des CO₂-Verdichters einschließlich seiner Zusatzeinrichtungen wie beispielsweise Antriebsturbine, Zwischenkühler, Ölsystem, etc. und die synergetische Nutzung des Hochdruckdesorbers für die Waschlösungen aus beiden Absorbern zu einer substantiellen Ersparnis führt.

Das Verfahren ermöglicht auch einen, nicht zur Erfindung gehörenden, separaten Betrieb einer Ammoniak-Anlage ohne Betrieb der Harnstoff-Anlage, da das Kohlendioxid wie bei der konventionellen Prozessführung dann an die Umgebung abgegeben werden kann. In diesem Fall braucht man den atmosphärischen Absorber nicht zu betreiben.

Gegenstand der vorliegenden Erfindung ist weiterhin eine Anlage zur Bereitstellung von Kohlendioxid für die Synthese von Harnstoff aus Ammoniak und Kohlendioxid, umfassend wenigstens einen Niederdruckabsorber, eine Einrichtung zur Zuführung eines ersten Ausgangsgasgemisches umfassend ein Kohlendioxid-haltiges Rauchgas zu diesem Niederdruckabsorber, eine Einrichtung zur Zuführung eines flüssigen Lösungsmittels auf Basis eines Ammoniak-Wasser-Gemisches zu diesem Niederdruckabsorber, einen Hochdruckabsorber, eine Einrichtung zur Zuführung eines zweiten Ausgangsgasgemisches umfassend ein Kohlendioxid-haltiges Synthesegas zu diesem Hochdruckabsorber, eine Einrichtung zur Zuführung eines flüssigen Lösungsmittels auf Basis eines Ammoniak-Wasser-Gemisches zu diesem Hochdruckabsorber, wenigstens eine Ausgangsleitung an dem Niederdruckabsorber für ein Abführen von mit Kohlendioxid beladenem Lösungsmittel aus dem Niederdruckabsorber, wenigstens eine Ausgangsleitung an dem Hochdruckabsorber für ein Abführen von mit Kohlendioxid beladenem Lösungsmittel aus dem Hochdruckabsorber, wobei die Anlage erfindungsgemäß weiterhin wenigstens eine Desorptionsvorrichtung umfasst, geeignet zum Austreiben von Kohlendioxid bei erhöhtem Druck aus dem mit Kohlendioxid beladenen Lösungsmittel.

Gemäß einer bevorzugten Weiterbildung der Erfindung sind der Niederdruckabsorber und der Hochdruckabsorber in einer gemeinsamen Apparatur angeordnet, welche eine Niederdruckzone und eine Hochdruckzone umfasst. Bei dieser Variante kann die Kohlendioxidwäsche sowohl des Rauchgases als auch diejenige des Synthesegases in einer gemeinsamen Apparatur mit der gleichen Waschlösung erfolgen.

Erfindungsgemäß umfasst die Anlage wenigstens eine erste Einrichtung, welche im Leitungsweg einer Leitung für aus der Desorptionsvorrichtung abgeführtes abgereichertes Lösungsmittel zwischen der Desorptionsvorrichtung und dem Hochdruckabsorber angeordnet ist, um das abgereicherte Lösungsmittel zu entspannen. Nach der Entspannung, in einer Turbine, kann das abgereicherte Lösungsmittel zurückgeführt werden zu dem Hochdruckabsorber.

Vorzugsweise umfasst die erfindungsgemäße Anlage weiterhin wenigstens eine zweite Turbine, welche im Leitungsweg einer Leitung für aus der Desorptionsvorrichtung abgeführtes abgereichertes Lösungsmittel zwischen der Desorptionsvorrichtung und dem Niederdruckabsorber und stromabwärts der ersten Turbine angeordnet ist, um das abgereicherte Lösungsmittel weiter zu entspannen, so dass der Druck soweit abgesenkt wird, dass das abgereicherte Lösungsmittel für die erneute Gaswäsche zurückgeführt werden kann zum Niederdruckabsorber.

Gemäß einer bevorzugten Weiterbildung der Erfindung münden die Ausgangsleitung des Niederdruckabsorbers und die Ausgangsleitung des Hochdruckabsorbers in eine gemeinsame Leitung für mit Kohlendioxid beladenes Lösungsmittel ein, welche zur Desorptionsvorrichtung führt. Auf diese Weise kann man die Lösungsmittelströme der mit Kohlendioxid beladenen Waschlösungen aus beiden Absorbern zusammenführen und gemeinsam der Desorptionsvorrichtung zuführen, um das Kohlendioxid auszutreiben.

Vorzugsweise befindet sich im Leitungsweg vom Niederdruckabsorber zu der Desorptionsvorrichtung wenigstens eine Einrichtung, vorzugsweise wenigstens eine Pumpe, um den Druck des beladenen Lösungsmittels zu erhöhen, da das Kohlendioxid bevorzugt bei einem erhöhten Druck ausgetrieben wird, der oberhalb des Drucks der Harnstoffsynthese liegt.

Weiterhin ist vorzugsweise im Leitungsweg vom Hochdruckabsorber zu der Desorptionsvorrichtung wenigstens eine Einrichtung, vorzugsweise eine Turbine angeordnet, um den Druck des beladenen Lösungsmittels zu verringern, damit dieser Strom des beladenen Lösungsmittels von Inerten befreit wird und etwa auf den gleichen Druck gebracht wird, wie der aus dem Niederdruckabsorber abgeführte Lösungsmittelstrom und anschließend beide beladenen Lösungsmittel zu einem gemeinsamen Strom vereint werden können, welcher der Desorptionsvorrichtung zugeführt wird.

Vorzugsweise ist weiterhin stromabwärts der Einmündung der Ausgangsleitung des Niederdruckabsorbers in die Ausgangsleitung des Hochdruckabsorbers in der gemeinsamen Leitung zur Desorptionsvorrichtung wenigstens eine Einrichtung, vorzugsweise eine Pumpe angeordnet ist, um den Druck des beladenen Lösungsmittels zu erhöhen. Die beiden Lösungsmittelströme des beladenen Lösungsmittels aus den beiden Absorbern können dann zunächst bei etwa gleichem Druck zusammengeführt werden und anschließend wird der Druck weiter erhöht, bevor der vereinte Lösungsmittelstrom der Desorptionsvorrichtung zugeführt wird.

Nachfolgend wird die vorliegende Erfindung anhand eines Ausführungsbeispiels unter Bezugnahme auf die beiliegende Zeichnung näher beschrieben. Dabei zeigt:

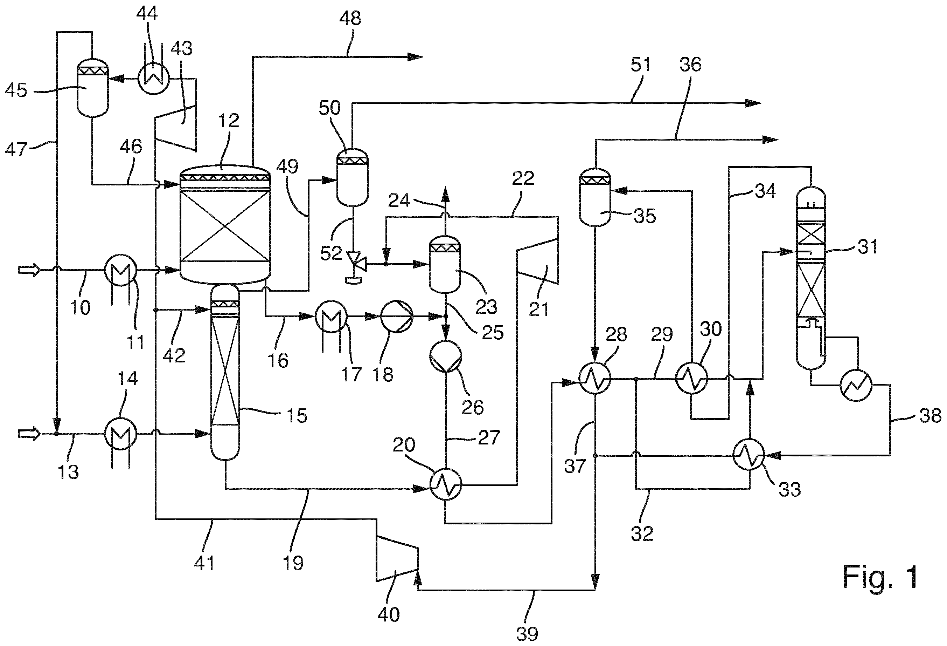

Figur 1 ein beispielhaftes Anlagenschema einer Anlage zur Bereitstellung von Kohlendioxid für die Harnstoffsynthese.

In der erfindungsgemäßen Anlage werden zwei Ausgangsgasströme zur Verfügung gestellt, welche jeweils unter anderem Kohlendioxid enthalten, nämlich zum einen ein Rauchgasstrom, welcher über eine Eingangsleitung 10 durch einen Wärmetauscher 11 strömt und einem etwa bei atmosphärischem Druck arbeitenden Niederdruckabsorber 12 zugeführt wird. In diesem Niederdruckabsorber 12 erfolgt eine Gaswäsche mittels einer verdünnten wässrigen Ammoniaklösung, um so u.a. Kohlendioxid aus dem Rauchgasstrom zu absorbieren.

Als zweiter Ausgangsgasstrom dient ein Synthesegasstrom, der über die Eingangsleitung 13 durch einen Wärmetauscher 14 strömt und dann einem Hochdruckabsorber 15 zugeführt wird, in dem ebenfalls eine Gaswäsche mit einer verdünnten wässrigen Ammoniaklösung erfolgt, um Kohlendioxid aus dem Synthesegas zu entfernen. Ein Merkmal der vorliegenden Erfindung besteht darin, dass zwei verschiedene Quellen für die Bereitstellung von Kohlendioxid genutzt werden, wobei in beiden Absorbern die Gaswäsche mit der gleichen Waschlösung erfolgen kann.

Nach der Gaswäsche in dem Niederdruckabsorber 12 verlässt ein an Kohlendioxid angereichertes Lösungsmittel (rich solvent) den Niederdruckabsorber über die Leitung 16, durchströmt einen Wärmetauscher 17 und wird dann mittels einer Pumpe 18 von einem etwa atmosphärischen Druck im Niederdruckabsorber auf einen erhöhten Druck von beispielsweise etwa 2 bar gebracht, damit diese Lösung des beladenen Lösungsmittels später mit der entsprechenden angereicherten Waschlösung aus dem Hochdruckabsorber vor der Pumpe 26 vereint werden kann. Das mit Kohlendioxid angereicherte Lösungsmittel verlässt den Hochdruckabsorber 15 über die Leitung 19, strömt dann durch einen Wärmetauscher 20, in welchem die Wärmeintegration stattfindet, wird beispielsweise über eine Turbine 21 entspannt und wird über die Leitung 22 einem Abscheider 23 zugeführt. Durch die Druckabsenkung können in dem Abscheider 23 für die Harnstoffsynthese inerte Gase wie beispielsweise Wasserstoff und Stickstoff aus dem Lösungsmittelstrom ausgegast und über die Leitung 24 abgeführt werden. Die Druckabsenkung des Lösungsmittelstroms aus dem Hochdruckabsorber 15 in der Turbine 21 kann auf einen Druck von beispielsweise etwa 2 bar erfolgen, so dass dieser Druck etwa mit demjenigen des Lösungsmittelstroms aus dem Niederdruckabsorber 12 übereinstimmt. Primär dient diese Druckabsenkung auf einen bestimmten Druck dem Zweck, die Inerte (hauptsächlich Wasserstoff und Stickstoff) auszugasen und die anderen Komponenten des Gemischs, insbesondere Ammoniak und Kohlendioxid, maximal zurückzuhalten. Dieser Zieldruck muss nicht unbedingt etwa dem Druck im Niederdruckabsorber entsprechen. An der Zweigstelle 24 a mündet dann die Leitung 16 aus dem Niederdruckabsorber 12 in die Leitung 25 aus dem Abscheider 23, so dass die beiden Lösungsmittelströme des beladenen Lösungsmittels aus beiden Absorbern vereinigt und anschließend mittels der Pumpe 26 auf einen erhöhten Druck gebracht werden können. Der vereinigte Strom aus dem beladenen Lösungsmittel strömt dann über die Leitung 27 durch den Wärmetauscher 20, wo ein Wärmetausch mit dem Medium in der von dem Hochdruckabsorber 15 kommenden Leitung 19 stattfindet.

Das auf einen erhöhten Druck gebrachte beladene Lösungsmittel strömt dann durch einen weiteren Wärmetauscher 28 und wird danach in zwei Teilströme aufgeteilt, wobei ein erster Teilstrom über die Leitung 29 durch einen Wärmetauscher 30 geführt und dann einer Desorptionsvorrichtung 31 zugeführt wird, während ein zweiter Teilstrom über die von der Leitung 29 abzweigende Zweigleitung 32 strömt, dann einen weiteren Wärmetauscher 33 durchströmt und dann wieder mit dem ersten Teilstrom zusammengeführt und der Desorptionsvorrichtung 31 zugeführt wird. Durch diese Aufteilung auf die beiden Teilströme kann die in den Produktströmen 34 und 38 der Desorptionsvorrichtung 31 enthaltene Wärmeenergie besser genutzt werden.

In der Desorptionsvorrichtung 31 wird das in dem Lösungsmittelstrom enthaltene Kohlendioxid bei einem erhöhten Druck desorbiert, so dass Kohlendioxid bei einem Druck vorliegt, der oberhalb des Synthesedrucks der Harnstoffsynthese liegt. Das desorbierte Kohlendioxid wird am Kopf der Desorptionsvorrichtung 31 über die Leitung 34 abgeführt, dann durch den Wärmetauscher 30 hindurchgeleitet und gelangt in einen Abscheider 35, in dem das Kondensat, welches während der Abkühlung in 30 entsteht, abgetrennt wird, während das Kohlendioxid, welches weiterhin Ammoniak und Wasser enthalten kann, über die vom Kopfbereich des Abscheiders 35 ausgehende Leitung 36 abgeführt und einer Harnstoffsyntheseanlage zugeführt werden kann.

Ein in dem Abscheider 35 abgetrennter Stoffstrom kann über die Leitung 37 durch den Wärmetauscher 28 strömen und dann weiter in die Leitung 39 strömen. Der an Kohlendioxid abgereicherte Lösungsmittelstrom (lean solvent) verlässt die Desorptionsvorrichtung 31 im Sumpfbereich und wird über die Leitung 38 durch den Wärmetauscher 33 geführt und dann mit dem Strom aus der Leitung 37 zusammengeführt. Dieser vereinte Strom des abgereicherten Lösungsmittels strömt dann über die Leitung 39 durch eine Turbine 40 und wird so auf einen niedrigeren Druck entspannt unter Nutzung der in dem Stoffstrom enthaltenen Energie und wird dann über die Leitung 41 als abgereichertes Lösungsmittel zu den beiden Absorbern zurückgeführt. Das abgereicherte Lösungsmittel weist nun einen geeigneten Druck auf, so dass ein Teilstrom aus der Leitung 41 abgezweigt und über die Leitung 42 als Waschlösung dem Hochdruckabsorber 15 zugeführt werden kann. Der übrige Teilstrom des abgereicherten Lösungsmittels strömt zunächst über die Leitung 41 weiter durch eine weitere Turbine 43 und wird dort auf einen niedrigeren Druck entspannt, strömt dann durch einen Wärmetauscher 44 und wird einem Abscheider 45 zugeführt, in dem eine Abtrennung von Gasen erfolgt, wobei anschließend dieser Teilstrom des abgereicherten Lösungsmittels über die Leitung 46 dem Niederdruckabsorber 12 zugeführt werden kann. In dem Abscheider 45 abgetrennte Gase können über die von dem Kopfbereich des Abscheiders 45 ausgehende Leitung 47 der Eingangsleitung 10 für das Rauchgas zugeführt werden, so dass eine Rückführung dieser abgetrennten Gase in die Gaswäsche gemeinsam mit dem frisch zugeführten Rauchgas erfolgen kann. Alternativ können die im Abscheider 45 abgetrennten Gase über die Leitung 47 direkt dem atmosphärischen Absorber zugeführt werden. Nach der Gaswäsche kann aus dem Kopfbereich des Niederdruckabsorbers 12 ein gereinigtes Rauchgas über die Ausgangsleitung 48 abgeführt werden.

Das im Hochdruckabsorber 15 gereinigte Synthesegas kann nach der Gaswäsche über die Leitung 49 einem weiteren Abscheider 50 zugeführt werden, wobei ein gereinigter Synthesegasstrom über die vom Kopfbereich des Abscheiders 50 ausgehende Ausgangsleitung 51 aus der Anlage abgeführt werden kann. Im Sumpfbereich des Weiteren Abscheiders 50 fällt nach Abtrennung des gereinigten Synthesegases ein Stoffstrom an, der über die Leitung 52 dem Abscheider 23 zugeführt wird und so mit dem Strom des beladenen Lösungsmittels vereint und in der zuvor beschriebenen Weise weiter behandelt und der Desorptionsvorrichtung 31 zugeführt werden kann.

Niederdruckabsorber 12 und Hochdruckabsorber 15 können wie in Figur 1 angedeutet in einer einzigen Apparatur untergebracht sein, welche zwei Zonen aufweist, nämlich eine Niederdruckzone und eine Hochdruckzone.

Ein wesentlicher Vorteil des erfindungsgemäßen Verfahrens ergibt sich daraus, dass das nach der Gaswäsche in den beiden Absorbern mit Kohlendioxid angereicherte Lösungsmittel nicht als solches der Harnstoffsynthese zugeführt wird, sondern mittels der Desorptionsvorrichtung 31 das Kohlendioxid aus dem Lösungsmittel ausgetrieben wird und bei für die Harnstoffsynthese geeignetem hohem Druck und vergleichsweise hoher Temperatur zur Verfügung gestellt wird. Das aus der Desorptionsvorrichtung abgeführte abgereicherte Lösungsmittel (lean solvent) kann zurückgeführt und somit erneut für den Absorptionsvorgang in den beiden Absorbern verwendet werden.

### Bezugszeichenliste

- 10: Eingangsleitung
- 11: Wärmetauscher
- 12: Niederdruckabsorber
- 13: Eingangsleitung
- 14: Wärmetauscher
- 15: Hochdruckabsorber
- 16: Leitung
- 17: Wärmetauscher
- 18: Pumpe
- 19: Leitung
- 20: Wärmetauscher
- 21: Turbine
- 22: Leitung
- 23: Abscheider
- 24: Ausgangsleitung
- 24 a: Zweigstelle
- 25: Leitung
- 26: Pumpe
- 27: Leitung
- 28: Wärmetauscher
- 29: Leitung
- 30: Wärmetauscher
- 31: Desorptionsvorrichtung
- 32: Zweigleitung
- 33: Wärmetauscher
- 34: Leitung
- 35: Abscheider
- 36: Leitung
- 37: Leitung
- 38: Leitung
- 39: Leitung
- 40: Turbine
- 41: Leitung
- 42: Leitung
- 43: Turbine
- 44: Wärmetauscher
- 45: Abscheider
- 46: Leitung
- 47: Rückführleitung
- 48: Ausgangsleitung
- 49: Leitung
- 50: Abscheider
- 51: Ausgangsleitung
- 52: Leitung

## Patentansprüche

1. Verfahren zur Bereitstellung von Kohlendioxid für die Synthese von Harnstoff aus Ammoniak und Kohlendioxid, bei dem als ein erstes Ausgangsgasgemisch ein Kohlendioxid-haltiges Rauchgas verwendet wird, wobei dieses erste Ausgangsgasgemisch einem Niederdruckabsorber (12) zugeführt wird, in dem Kohlendioxid aus dem ersten Ausgangsgasgemisch in einem flüssigen Lösungsmittel auf Basis eines Ammoniak-Wasser-Gemisches absorbiert wird, wobei weiterhin als ein zweites Ausgangsgasgemisch ein Kohlendioxid-haltiges Synthesegas verwendet wird und dieses zweite Ausgangsgasgemisch einem Hochdruckabsorber (15) zugeführt wird, in dem Kohlendioxid aus diesem zweiten Ausgangsgasgemisch in einem flüssigen Lösungsmittel auf Basis eines Ammoniak-Wasser-Gemisches bei erhöhtem Druck absorbiert wird, **dadurch gekennzeichnet, dass** für den Absorptionsschritt in dem Niederdruckabsorber (12) und für den Absorptionsschritt in dem Hochdruckabsorber (15) das gleiche an Kohlendioxid abgereicherte flüssige Lösungsmittel verwendet wird, welches in wenigstens zwei parallelen Teilströmen dem Hochdruckabsorber und dem Niederdruckabsorber zugeführt wird.

2. Verfahren nach Anspruch 1, **dadurch gekennzeichnet, dass** ein erster aus dem Niederdruckabsorber (12) austretender mit Kohlendioxid angereicherter Lösungsmittelstrom (16) und ein zweiter aus dem Hochdruckabsorber (15) austretender mit Kohlendioxid angereicherter Lösungsmittelstrom (19) anschließend zu einem gemeinsamen Lösungsmittelstrom (27) vereint werden.

3. Verfahren nach Anspruch 1 oder 2, **dadurch gekennzeichnet, dass** aus einem ersten, aus dem Niederdruckabsorber (12) austretenden mit Kohlendioxid angereicherten Lösungsmittelstrom (16) oder aus einem zweiten, aus dem Hochdruckabsorber (15) austretenden mit Kohlendioxid angereicherten Lösungsmittelstrom (19) oder aus einem vereinten gemeinsamen Lösungsmittelstrom (27) aus Niederdruckabsorber und Hochdruckabsorber anschließend in einer Desorptionsvorrichtung (31) Kohlendioxid desorbiert wird.

4. Verfahren nach Anspruch 3, **dadurch gekennzeichnet, dass** das mit Kohlendioxid angereicherte Lösungsmittel auf einen Druck oberhalb des Harnstoffsynthesedrucks komprimiert wird, in einer Desorptionsvorrichtung (31) das Kohlendioxid bei einem Druck, welcher oberhalb des Harnstoffsynthesedrucks liegt aus dem Lösungsmittel desorbiert wird und dann das desorbierte Kohlendioxid einer Harnstoffsyntheseeinheit zugeführt wird.

5. Verfahren nach Anspruch 2, **dadurch gekennzeichnet, dass** das mit Kohlendioxid aus dem Rauchgas angereicherte Lösungsmittel aus dem Niederdruckabsorber (12) zunächst auf einen erhöhten Druck gebracht wird und anschließend mit dem mit Kohlendioxid aus dem Synthesegas angereicherten Lösungsmittel aus dem Hochdruckabsorber (15) vereint wird.

6. Verfahren nach einem der Ansprüche 1 bis 4, **dadurch gekennzeichnet, dass** der zweite aus dem Hochdruckabsorber (15) austretende mit Kohlendioxid angereicherte Lösungsmittelstrom anschließend abgekühlt und danach entspannt wird, wobei in dem Lösungsmittel absorbierte Gase, die von Kohlendioxid verschieden sind, insbesondere Stickstoff und/oder Wasserstoff aus dem Lösungsmittel ausgegast und abgetrennt werden.

7. Verfahren nach einem der Ansprüche 2 bis 6, **dadurch gekennzeichnet, dass** der vereinte Lösungsmittelstrom aus Hochdruckabsorber (15) und Niederdruckabsorber (12) nach dem Vereinen auf einen höheren Druck gebracht wird, wobei die Druckerhöhung vorzugsweise mittels einer Pumpe (26) im flüssigen Zustand erfolgt.

8. Verfahren nach einem der Ansprüche 3 bis 7, **dadurch gekennzeichnet, dass** von Kohlendioxid abgereichertes Lösungsmittel aus der Desorptionsvorrichtung (31) wenigstens teilweise zu dem Niederdruckabsorber (12) und/oder wenigstens teilweise zu dem Hochdruckabsorber (15) zurückgeführt wird.

9. Verfahren nach einem der Ansprüche 3 bis 8, **dadurch gekennzeichnet, dass** der vereinte Lösungsmittelstrom aus dem Niederdruckabsorber (12) und dem Hochdruckabsorber (15), bevor dieser der Desorptionsvorrichtung (31) zugeführt wird, in wenigstens zwei Teilströme (29, 32) aufgeteilt wird, von denen der erste Teilstrom (29) einem ersten Wärmetauscher (30) zugeführt wird und der zweite Teilstrom (32) einem zweiten Wärmetauscher (33) zugeführt wird, wobei nach Durchströmen der beiden Wärmetauscher (30, 33) die beiden Teilströme wieder vereint und dann der Desorptionsvorrichtung (31) zugeführt werden.

10. Verfahren nach einem der Ansprüche 1 bis 9, **dadurch gekennzeichnet, dass** als Lösungsmittel für die Kohlendioxidabsorption in den beiden Absorbern (12, 15) eine verdünnte wässrige Ammoniaklösung verwendet wird.

11. Verfahren nach Anspruch 10, **dadurch gekennzeichnet, dass** eine verdünnte wässrige Ammoniaklösung mit einem Ammoniakgehalt von 5 bis 30 Gew.-%, bevorzugt 10 bis 25 Gew.-%, noch bevorzugter 15 bis 20 Gew.-% verwendet wird.

12. Verfahren nach einem der Ansprüche 1 bis 11, **dadurch gekennzeichnet, dass** das Rauchgas vor dem Schritt der Absorption in dem Niederdruckabsorber (12) erwärmt oder gekühlt wird.

13. Verfahren nach einem der Ansprüche 1 bis 12, **dadurch gekennzeichnet, dass** das Rauchgas vor dem Schritt der Absorption in dem Niederdruckabsorber (12) zur Entfernung von Flugasche oder Rußpartikeln filtriert oder einer Nasswäsche unterzogen wird.

14. Verfahren nach einem der Ansprüche 1 bis 13, **dadurch gekennzeichnet, dass** aus dem Kopfbereich des Niederdruckabsorbers (12) ein von Kohlendioxid gereinigtes Rauchgas abgeführt wird und/oder aus dem Kopfbereich des Hochdruckabsorbers (15) ein gereinigtes Synthesegas abgeführt wird.

15. Anlage zur Bereitstellung von Kohlendioxid für die Synthese von Harnstoff aus Ammoniak und Kohlendioxid, umfassend wenigstens einen Niederdruckabsorber (12), eine Einrichtung (10) zur Zuführung eines ersten Ausgangsgasgemisches umfassend ein Kohlendioxid-haltiges Rauchgas zu diesem Niederdruckabsorber (12), eine Einrichtung (46) zur Zuführung eines flüssigen Lösungsmittels auf Basis eines Ammoniak-Wasser-Gemisches zu diesem Niederdruckabsorber (12), einen Hochdruckabsorber (15), eine Einrichtung (13) zur Zuführung eines zweiten Ausgangsgasgemisches umfassend ein Kohlendioxid-haltiges Synthesegas zu diesem Hochdruckabsorber (15), eine Einrichtung (42) zur Zuführung eines flüssigen Lösungsmittels auf Basis eines Ammoniak-Wasser-Gemisches zu diesem Hochdruckabsorber (15), wenigstens eine Ausgangsleitung (16) an dem Niederdruckabsorber (12) für ein Abführen von mit Kohlendioxid beladenem Lösungsmittel aus dem Niederdruckabsorber, wenigstens eine Ausgangsleitung (19) an dem Hochdruckabsorber (15) für ein Abführen von mit Kohlendioxid beladenem Lösungsmittel aus dem Hochdruckabsorber, **dadurch gekennzeichnet, dass** die Anlage weiterhin wenigstens eine Desorptionsvorrichtung (31) umfasst, geeignet zum Austreiben von Kohlendioxid bei erhöhtem Druck aus dem mit Kohlendioxid beladenen Lösungsmittel, wobei die Anlage wenigstens eine erste Turbine (40) umfasst, welche im Leitungsweg einer Leitung (39) für aus der Desorptionsvorrichtung (31) abgeführtes abgereichertes Lösungsmittel zwischen der Desorptionsvorrichtung (31) und dem Hochdruckabsorber (15) angeordnet ist, um das abgereicherte Lösungsmittel zu entspannen und gespeicherte mechanische Energie zurückzugewinnen.

16. Anlage nach Anspruch 15, **dadurch gekennzeichnet, dass** diese eine Einrichtung (47) zur Zuführung eines Gasgemisches aus einem Abscheider (45) zu dem Niederdruckabsorber (12) umfasst.

17. Anlage nach Anspruch 15 oder 16, **dadurch gekennzeichnet, dass** der Niederdruckabsorber (12) und der Hochdruckabsorber (15) in einer gemeinsamen Apparatur angeordnet sind, welche eine Niederdruckzone und eine Hochdruckzone umfasst.

18. Anlage nach einem der Ansprüche 15 bis 17, **dadurch gekennzeichnet, dass** die Ausgangsleitung (16) des Niederdruckabsorbers (12) und die Ausgangsleitung (19) des Hochdruckabsorbers (15) in eine gemeinsame Leitung (27) für mit Kohlendioxid beladenes Lösungsmittel einmünden, welche zur Desorptionsvorrichtung (31) führt.

## Claims

1. Process for providing carbon dioxide for the synthesis of urea from ammonia and carbon dioxide, in which a carbon dioxide-containing flue gas is used as a first starting gas mixture, where this first starting gas mixture is supplied to a low-pressure absorber (12) in which carbon dioxide is absorbed from the first starting gas mixture in a liquid solvent based on an ammonia-water mixture, where a carbon dioxide-containing synthesis gas is also used as a second starting gas mixture and this second starting gas mixture is supplied to a high-pressure absorber (15) in which carbon dioxide is absorbed from this second starting gas mixture in a liquid solvent based on an ammonia-water mixture at elevated pressure, **characterized in that** the same carbon dioxide-depleted liquid solvent is used for the absorption step in the low-pressure absorber (12) and for the absorption step in the high-pressure absorber (15) and is supplied to the high-pressure absorber and the low-pressure absorber in at least two parallel substreams.

2. Process according to Claim 1, **characterized in that** a first carbon dioxide-enriched solvent stream (16) exiting from the low-pressure absorber (12) and a second carbon dioxide-enriched solvent stream (19) exiting from the high-pressure absorber (15) are subsequently combined to give a common solvent stream (27) .

3. Process according to Claim 1 or 2, **characterized in that** carbon dioxide is subsequently desorbed in a desorption apparatus (31) from a first carbon dioxide-enriched solvent stream (16) exiting from the low-pressure absorber (12) or from a second carbon dioxide-enriched solvent stream (19) exiting from the high-pressure absorber (15) or from a combined common solvent stream (27) from low-pressure absorber and high-pressure absorber.

4. Process according to Claim 3, **characterized in that** the carbon dioxide-enriched solvent is compressed to a pressure above the urea synthesis pressure, the carbon dioxide is desorbed from the solvent in a desorption apparatus (31) at a pressure above the urea synthesis pressure, and then the desorbed carbon dioxide is sent to a urea synthesis unit.

5. Process according to Claim 2, **characterized in that** the solvent enriched with carbon dioxide from the flue gas from the low-pressure absorber (12) is first brought to elevated pressure and then combined with the solvent enriched with carbon dioxide from the synthesis gas from the high-pressure absorber (15).

6. Process according to any of Claims 1 to 4, **characterized in that** the second carbon dioxide-enriched solvent stream exiting from the high-pressure absorber (15) is subsequently cooled and then expanded, with outgassing and separation of gases other than carbon dioxide absorbed in the solvent, especially nitrogen and/or hydrogen, from the solvent.

7. Process according to any of Claims 2 to 6, **characterized in that** the combined solvent stream from high-pressure absorber (15) and low-pressure absorber (12), after combination, is brought to a higher pressure, where the pressure increase is preferably effected in the liquid state by means of a pump (26).

8. Process according to any of Claims 3 to 7, **characterized in that** carbon dioxide-depleted solvent from the desorption apparatus (31) is recycled at least partly to the low-pressure absorber (12) and/or at least partly to the high-pressure absorber (15).

9. Process according to any of Claims 3 to 8, **characterized in that** the combined solvent stream from the low-pressure absorber (12) and the high-pressure absorber (15), before being supplied to the desorption apparatus (31), is divided into at least two substreams (29, 32), of which the first substream (29) is supplied to a first heat exchanger (30) and the second substream (32) is supplied to a second heat exchanger (33), and, after flowing through the two heat exchangers (30, 33), the two substreams are combined again and then supplied to the desorption apparatus (31).

10. Process according to any of Claims 1 to 9, **characterized in that** the solvent used for the carbon dioxide absorption in the two absorbers (12, 15) is a dilute aqueous ammonia solution.

11. Process according to Claim 10, **characterized in that** a dilute aqueous ammonia solution having an ammonia content of 5% to 30% by weight, preferably 10% to 25% by weight, more preferably 15% to 20% by weight, is used.

12. Process according to any of Claims 1 to 11, **characterized in that** the flue gas is heated or cooled prior to the step of absorption in the low-pressure absorber (12).

13. Process according to any of Claims 1 to 12, **characterized in that** the flue gas is filtered or subjected to wet scrubbing for removal of fly ash or soot particles prior to the step of absorption in the low-pressure absorber (12).

14. Process according to any of Claims 1 to 13, **characterized in that** a carbon dioxide-purged flue gas is removed from the top region of the low-pressure absorber (12) and/or a cleaned synthesis gas is removed from the top region of the high-pressure absorber (15).

15. Plant for providing carbon dioxide for the synthesis of urea from ammonia and carbon dioxide, comprising at least one low-pressure absorber (12), a device (10) for supply of a first starting gas mixture comprising a carbon dioxide-containing flue gas to this low-pressure absorber (12), a device (46) for supply of a liquid solvent based on an ammonia-water mixture to this low-pressure absorber (12), a high-pressure absorber (15), a device (13) for supply of a second starting gas mixture comprising a carbon dioxide-containing synthesis gas to this high-pressure absorber (15), a device (42) for supply of a liquid solvent based on an ammonia-water mixture to this high-pressure absorber (15), at least one exit conduit (16) on the low-pressure absorber (12) for removal of carbon dioxide-laden solvent from the low-pressure absorber, and at least one exit conduit (19) on the high-pressure absorber (15) for removal of carbon dioxide-laden solvent from the high-pressure absorber, **characterized in that** the plant further comprises at least one desorption apparatus (31) suitable for driving carbon dioxide at elevated pressure out of the carbon dioxide-laden solvent, where the plant comprises at least one first turbine (40) disposed between the desorption apparatus (31) and the high-pressure absorber (15) in the conduit pathway of a conduit (29) for depleted solvent removed from the desorption apparatus (31), in order to expand the depleted solvent and recover stored mechanical energy.

16. Plant according to Claim 15, **characterized in that** it comprises a device (47) for supply of a gas mixture from a separator (45) to the low-pressure absorber (12) .

17. Plant according to Claim 15 or 16, **characterized in that** the low-pressure absorber (12) and the high-pressure absorber (15) are disposed in a common apparatus comprising a low-pressure zone and a high-pressure zone.

18. Plant according to any of Claims 15 to 17, **characterized in that** the exit conduit (16) from the low-pressure absorber (12) and the exit conduit (19) from the high-pressure absorber (15) open into a common conduit (27) for carbon dioxide-laden solvent that leads to the desorption apparatus (31).

## Revendications

1. Procédé pour la fourniture de dioxyde de carbone destiné à la synthèse de l'urée à partir d'ammoniac et de dioxyde de carbone, dans lequel on utilise en tant qu'un premier mélange de gaz de départ un gaz de fumée contenant du dioxyde de carbone, ce premier mélange de gaz de départ étant envoyé à un absorbeur basse pression (12), dans lequel du dioxyde de carbone provenant du premier mélange de gaz de départ est absorbé dans un solvant liquide à base d'un mélange ammoniac-eau, un gaz de synthèse contenant du dioxyde de carbone étant en outre utilisé en tant qu'un second mélange de gaz de départ et ce second mélange de gaz de départ étant envoyé à un absorbeur haute pression (15), dans lequel du dioxyde de carbone provenant de ce second mélange de gaz de départ est absorbé sous pression élevée dans un solvant liquide à base d'un mélange ammoniac-eau, **caractérisé en ce que** pour l'étape d'absorption dans l'absorbeur basse pression (12) et pour l'étape d'absorption dans l'absorbeur haute pression (15), on utilise le même solvant liquide appauvri en dioxyde de carbone, qui est envoyé en au moins deux courants partiels parallèles à l'absorbeur haute pression et à l'absorbeur basse pression.

2. Procédé selon la revendication 1, **caractérisé en ce qu'**ensuite on réunit en un courant de solvant commun (27) un premier courant de solvant (16) enrichi en dioxyde de carbone, sortant de l'absorbeur basse pression (12), et un second courant de solvant (19) enrichi en dioxyde de carbone, sortant de l'absorbeur haute pression (15).

3. Procédé selon la revendication 1 ou 2, **caractérisé en ce qu'**à partir d'un premier courant de solvant (16) enrichi en dioxyde de carbone, sortant de l'absorbeur basse pression (12), ou à partir d'un second courant de solvant (19) enrichi en dioxyde de carbone, sortant de l'absorbeur haute pression (15) ou à partir d'un courant de solvant commun (27) réuni, provenant de l'absorbeur basse pression et de l'absorbeur haute pression, du dioxyde de carbone est ensuite désorbé dans un dispositif de désorption (31).

4. Procédé selon la revendication 3, **caractérisé en ce que** le solvant enrichi en dioxyde de carbone est comprimé jusqu'à une pression supérieure à la pression de synthèse de l'urée, dans un dispositif de désorption (31) le dioxyde de carbone est désorbé à partir du solvant sous une pression qui se situe au-dessus de la pression de synthèse de l'urée et le dioxyde de carbone désorbé est ensuite envoyé à une unité de synthèse de l'urée.

5. Procédé selon la revendication 2, **caractérisé en ce que** le solvant provenant de l'absorbeur (12) basse pression, enrichi en dioxyde de carbone provenant du gaz de fumée, est d'abord mis sous une pression élevée et ensuite est réuni avec le solvant provenant de l'absorbeur (15) haute pression, enrichi en dioxyde de carbone provenant du gaz de synthèse.

6. Procédé selon l'une quelconque des revendications 1 à 4, **caractérisé en ce que** le second courant de solvant enrichi en dioxyde de carbone, sortant de l'absorbeur haute pression (15), est ensuite refroidi, puis détendu, les gaz absorbés dans le solvant, qui sont différents du dioxyde de carbone, en particulier l'azote et/ou l'hydrogène, étant extraits du solvant et séparés.

7. Procédé selon l'une quelconque des revendications 2 à 6, **caractérisé en ce que** le courant de solvant réuni provenant de l'absorbeur haute pression (15) et de l'absorbeur basse pression (12) est, après la réunion, mis sous une pression plus élevée, l'élévation de pression s'effectuant à l'état liquide de préférence au moyen d'une pompe (26).

8. Procédé selon l'une quelconque des revendications 3 à 7, **caractérisé en ce que** le solvant appauvri en dioxyde de carbone, provenant du dispositif de désorption (31), est renvoyé au moins en partie à l'absorbeur basse pression (12) et/ou au moins en partie à l'absorbeur haute pression (15).

9. Procédé selon l'une quelconque des revendications 3 à 8, **caractérisé en ce que** le courant de solvant réuni provenant de l'absorbeur basse pression (12) et de l'absorbeur haute pression (15) est scindé, avant d'être envoyé au dispositif de désorption (31), en au moins deux courants partiels (29, 32), dont le premier courant partiel (29) est envoyé à un premier échangeur de chaleur (30) et le deuxième courant partiel (32) est envoyé à un deuxième échangeur de chaleur (33), les deux courants partiels, après avoir traversé les deux échangeurs de chaleur (30, 33), étant de nouveau réunis et ensuite envoyés au dispositif de désorption (31).

10. Procédé selon l'une quelconque des revendications 1 à 9, **caractérisé en ce que** pour l'absorption du dioxyde de carbone dans les deux absorbeurs (12, 15) on utilise comme solvant une solution aqueuse d'ammoniac diluée.

11. Procédé selon la revendication 10, **caractérisé en ce qu'**on utilise une solution aqueuse d'ammoniac diluée ayant une teneur en ammoniac de 5 à 30 % en poids, de préférence 10 à 25 % en poids, encore mieux 15 à 20 % en poids.

12. Procédé selon l'une quelconque des revendications 1 à 11, **caractérisé en ce que** le gaz de fumée est chauffé ou refroidi avant l'étape de l'absorption dans l'absorbeur basse pression (12).

13. Procédé selon l'une quelconque des revendications 1 à 12, **caractérisé en ce qu'**avant l'étape de l'absorption dans l'absorbeur basse pression (12) le gaz de fumée est filtré ou soumis à un lavage par voie humide, pour l'élimination de cendres volantes ou de particules de noir de fumée.

14. Procédé selon l'une quelconque des revendications 1 à 13, **caractérisé en ce qu'**un gaz de fumée épuré de dioxyde de carbone est évacué de la zone de tête de l'absorbeur basse pression (12) et/ou un gaz de synthèse épuré est évacué de la zone de tête de l'absorbeur haute pression (15).

15. Installation destinée à la fourniture de dioxyde de carbone destiné à la synthèse de l'urée à partir d'ammoniac et de dioxyde de carbone, comprenant au moins un absorbeur basse pression (12), un dispositif (10) destiné à l'envoi d'un premier mélange de gaz de départ, comprenant un gaz de fumée contenant du dioxyde de carbone, à cet absorbeur basse pression (12), un dispositif (46) destiné à l'envoi d'un solvant liquide à base d'un mélange ammoniac-eau à cet absorbeur basse pression (12), un absorbeur haute pression (15), un dispositif (13) destiné à l'envoi d'un second mélange de gaz de départ, comprenant un gaz de synthèse contenant du dioxyde de carbone, à cet absorbeur haute pression (15), un dispositif (42) destiné à l'envoi d'un solvant liquide à base d'un mélange ammoniac-eau à cet absorbeur haute pression (15), au moins un conduit de sortie (16) raccordé à l'absorbeur basse pression (12) pour une évacuation de solvant chargé de dioxyde de carbone à partir de l'absorbeur basse pression, au moins un conduit de sortie (19) raccordé à l'absorbeur basse pression (15) pour une évacuation de solvant chargé de dioxyde de carbone à partir de l'absorbeur haute pression, **caractérisé en ce que** l'installation comprend en outre au moins un dispositif de désorption (31), approprié à l'extraction de dioxyde de carbone sous pression élevée à partir du solvant chargé de dioxyde de carbone, l'installation comprenant au moins une première turbine (40) qui est disposée entre le dispositif de désorption (31) et l'absorbeur haute pression (15) sur le trajet d'un conduit (39) pour solvant appauvri, évacué du dispositif de désorption (31), afin de détendre le solvant appauvri et de récupérer de l'énergie mécanique emmagasinée.

16. Installation selon la revendication 15, **caractérisée en ce qu'**elle comprend un dispositif (47) destiné à l'envoi d'un mélange de gaz à partir d'un séparateur (45) vers l'absorbeur basse pression (12).

17. Installation selon la revendication 15 ou 16, **caractérisée en ce que** l'absorbeur basse pression (12) et l'absorbeur haute pression (15) sont disposés dans un appareil commun qui comprend une zone basse pression et une zone haute pression.

18. Installation selon l'une quelconque des revendication 15 à 17, **caractérisée en ce que** le conduit de sortie (16) de l'absorbeur basse pression (12) et le conduit de sortie (19) de l'absorbeur haute pression (15) débouchent dans un conduit commun (27) pour solvant chargé de dioxyde de carbone, qui mène au dispositif de désorption (31).
